# EUROPÄISCHE PATENTSCHRIFT

(11) **EP 2 996 750 B1**
(45) Veröffentlichungstag und Bekanntmachung des Hinweises auf die Patenterteilung: **19.09.2018**
(21) Anmeldenummer: 14733974.1
(22) Anmeldetag: 13.05.2014
(51) Int. Cl.: A61M 16/00, A61M 16/06

(54) **VORRICHTUNG ZUR ATEMSPENDE IN NOTFÄLLEN**
DEVICE FOR ARTIFICIAL RESPIRATION IN EMERGENCIES
DISPOSITIF D'ASSISTANCE RESPIRATOIRE D'URGENCE

(30) Priorität: 13.05.2013 DE 102013208776; 13.11.2013 DE 102013223125
(43) Veröffentlichungstag der Anmeldung: 23.03.2016
(73) Patentinhaber: Karl Küfner GmbH & Co. KG, 72461 Albstadt-Truchtelfingen (DE)
(72) Erfinder: BRONNER, Rolf, 76532 Baden-Baden (DE); HILLER, Joachim, 70353 Göppingen (DE); KAZ, Till, 70186 Stuttgart (DE)
(74) Vertreter: Geitz Truckenmüller Lucht Christ
(86) Internationale Anmeldenummer: PCT/DE2014/100166
(87) Internationale Veröffentlichungsnummer: WO 2014/183747

(56) Entgegenhaltungen:
- DE-A1- 2 402 192
- GB-A- 2 379 612
- US-A1- 2003 192 547

## Beschreibung

Die Erfindung geht aus von einer Vorrichtung zur Atemspende in Notfällen.

Bei einem Unfall oder sonstigen medizinischen Notfall sind die Betroffenen durch Ersthelfer im Rahmen der ersten Hilfe zu versorgen bis professionelle Rettungskräfte eintreffen. Dabei sind Ersthelfer Personen, die als zufällige Helfer bei einem Unfall oder einem medizinischen Notfall lebensrettende Sofortmaßnahmen einleiten können. Hat bei der von einem Unfall oder medizinischen Notfall betroffenen Person die körpereigene Atmung ganz oder teilweise ausgesetzt oder liegt ein Kreislaufstillstand vor, so müssen Maßnahmen zur Wiederbelebung und zur Beendigung des Atemstillstands durchgeführt werden. Hierzu zählt die Herz-Lungen-Wiederbelebung. Sind die Atemwege blockiert oder verlegt, so müssen die Atemwege der betroffenen Person zunächst frei gemacht werden. Gegebenenfalls muss die Person durch einen Hilfeleistenden beamtet werden. Bei einem Kreislaufstillstand muss eine Herzdruckmassage durchgeführt werden. Das Ziel dieser Maßnahmen ist die Versorgung lebenswichtiger Organe der betroffenen Person mit Sauerstoff.

Im folgenden wird die Person, die sich in einem medizinischen Notfall befindet als Person bezeichnet. Die Person, die erste Hilfe leistet, wird im folgenden als Hilfeleistender bezeichnet. Maßnahmen zur Reanimation sind im folgenden solche, die geeignet sind, in einem gewissen Umfang einen Kreislauf aufrecht zu erhalten, um die wichtigsten Organe zu versorgen bis professionelle Rettungskräfte eintreffen.

Die einfachste Form der Beatmung ist die Atemspende durch Mund-zu-Mund-Beatmung. Dabei wird bei überstrecktem Kopf des Unfall- oder Notfallopfers dessen Nase verschlossen und über den Mund Luft insuffliert. Hierbei muss zu großer Druck vermieden werden um eine Aspiration, nämlich das Eindringen von Speichel, Flüssigkeit oder sonstigem Material in die Atemwege zu verhindern. Ein adäquates Volumen ist erreicht, wenn ein Heben des Brustkorbes des Unfall- oder Notfallopfers zu erkennen ist.

Potentielle Ersthelfer verfügen häufig über keine oder unzureichende Kenntnisse in der ersten Hilfe. Aus Angst vor Fehlern aufgrund dieser unzureichenden Kenntnisse wird häufig keine oder unzureichende erste Hilfe geleistet. Darüber hinaus wird von potentiellen Ersthelfern häufig ein Atemstillstand oder ein Kreislaufstillstand nicht erkannt. Als Folge werden notwendige Hilfeleistungen nicht eingeleitet. Bei einer Mund-zu-Mund-Beatmung kommt der Hilfeleistende in direkten Kontakt mit Körperflüssigkeiten des Unfall- oder Notfallopfers mit der Folge der möglichen Übertragung von Krankheiten. Es besteht daher häufig eine Hemmschwelle.

Durch bekannte Beatmungshilfen wie Masken oder Folien, welche auf Mund und Nase eines Unfall- oder Notfallopfers aufsetzbar sind, wird eine direkte Berührung zwischen Unfall- oder Notfallopfer einerseits und Hilfeleistendem andererseits vermieden. Dadurch wird das Infektionsrisiko gemindert. Bekannte Beatmungshilfen sind mit einem Mundstück ausgestattet, über das der Hilfeleistende seine eigene Atemluft einbläst. Die ausgeatmete Luft des Unfall- oder Notfallopfers entweicht bei erfolgreicher Beatmung über die Maske und kommt nicht zum Hilfeleistenden zurück. Probleme bei der Verwendung bekannter Beatmungshilfen sind das Auftreten von Leckagen, ein erhöhter Beatmungswiderstand und gegebenenfalls eine unzureichende Kenntnis des Hilfeleistenden über die Beatmungszeit sowie die Menge der erforderlichen Inspirationsluft. Die Probleme der unzureichenden Kenntnis des Hilfeleistenden im Bezug auf die Herzdruckmassage bleiben auch bei der Verwendung bekannter Beatmungshilfen bestehen.

Eine derartige Beatmungshilfe ist aus der DE 24 02 192 A1 bekannt. Die Beatmungshilfe ist mit einer Mund und Nase eines Patienten überdeckenden Maske, einem an der Maske angebrachten Kolbenventil, einem Mundstück für einen Hilfeleistenden und einer Handpumpe ausgestattet. Der Hilfeleistende erhält jedoch keinerlei Hilfestellung oder Anweisung für die Zufuhr von Atemluft oder die Durchführung einer Herzdruckmassage.

Die GB 2 379 612 A offenbart eine Beatmungshilfe mit einer auf Mund und Nase eines Patienten aufsetzbaren Maske, mit einem Einlass zum Zuführen von Atemluft und einem Kontrollventil zwischen der Maske und dem Einlass. Über das Ventil wird die aus dem Patienten bei der Beatmung ausströmende Luft einem Gas-Indikator zugeführt, der den Anteil an Kohlendioxid in der aus dem Patienten ausströmenden Luft erfasst und anzeigt. Der Anstieg des Anteils an Kohlendioxid in der aus dem Patienten ausströmenden Luft ist ein Indiz für eine Spontanatmung des Patienten. Ferner überwacht ein Sensor den Luftstrom, um festzustellen, ob die Atemwege der Patienten verlegt sind. Der Hilfeleistende erhält jedoch keinerlei Hilfestellung oder Anweisung für die Durchführung einer Herzdruckmassage.

Die US 2003/0192547 A1 offenbart eine Beatmungshilfe mit einer Maske für das Gesicht eines Patienten, einem Ventilsystem, über das Atemluft der Maske zugeführt und aus der Maske wieder abgeführt wird. Zusätzlich ist die Beatmungshilfe mit einem Metronom ausgestattet, das ein periodisches akustisches Signal erzeugt. Dieses Signal soll einem Hilfeleistenden dazu dienen, eine Herzdruckmassage mit einer durch das Metronom vorgegebenen Frequenz auszuführen. Nachteilig ist jedoch, dass der Hilfeleistenden keine Hilfestellung und Anweisung bei der Eindringtiefe der Herzdruckmassage erhält.

Der Erfindung liegt die Aufgabe zugrunde, eine Vorrichtung zur Atemspende in Notfällen zur Verfügung zu stellen, bei denen ein Hilfeleistender einem Unfall- oder Notfallopfer Atemluft zuführen kann, ohne mit dessen Nase und Mund in direkten Körperkontakt zu treten, und bei dem dem Hilfeleistenden die Zufuhr von Atemluft sowie die Herzdruckmassage erleichtert wird, auch dann, wenn dieser keine oder unzureichende Kenntnisse in erster Hilfe besitzt.

Diese Aufgabe wird durch eine Vorrichtung mit den Merkmalen des Anspruchs 1 gelöst. Die erfindungsgemäße Vorrichtung zeichnet sich dadurch aus, dass zwischen einer auf Nase und Mund eines Unfall- oder Notfallopfers aufsetzbaren Beatmungsmaske einerseits und einem Mundstück für den Hilfeleistenden andererseits ein Störmungstubus angeordnet ist. Dieser bildet einen für die der Person zugeführte oder von dieser ausströmenden Luft durchgängigen Strömungskanal. An dem Strömungstubus ist ein Strömungssensor angeordnet, der in den Strömungskanal hineinragt. Der Strömungssensor wird von der durch den Strömungskanal geleiteten Luft umströmt und erfasst dabei den Masse- oder Volumenstrom eines durch den Strömungskanal strömenden Gases. Dabei entspricht der Volumenstrom eines durch den Strömungstubus strömenden Gases einem Volumen, das pro Zeiteinheit einen vorgegebenen Querschnitt des Strömungstubus passiert. Der Massestrom oder Massedurchfluss eines durch den Strömungstubus strömenden Gases entspricht einer Masse, die pro Zeiteinheit einen vorgegebenen Querschnitt des Strömungstubus passiert.

Der Volumenstrom oder Durchfluss bezogen auf die der Person zugeführte Atemluft wird auch als inspiratorischer Flow bezeichnet. Der Volumenstrom oder Durchfluss bezogen auf die von der Person ausströmenden Luft wird auch als expiratorischer Flow bezeichnet.

Aus dem Masse- oder Volumenstrom kann auch eine Änderungsrate des Masse- oder Volumenstroms bestimmt werden.

Die Beatmungsmaske liegt auf dem Gesicht der Person auf. Sie kann gegebenenfalls mit einem Band oder einem Gurt am Kopf der Person befestigt werden, so dass sie ihre Position beibehält. Aufgrund eines weichen und elastischen Randes liegt die Beatmungsmaske im wesentlichen dichtend auf dem Gesicht der Person auf. Dies führt dazu, dass die aus Nase oder Mund der Person ausströmende Luft im wesentlichen in dem Strömungstubus gelangt und durch diesen hindurch strömt. Dabei passiert sie den Strömungssensor, so dass der Masse- oder Volumenstrom der strömenden Luft erfasst wird. Darüber hinaus gelangt die von einem Hilfeleistenden über das Mundstück zugeführte Luft durch den Strömungstubus in die Beatmungsmaske und von dort in Nase und/ oder Mund der Person. Der Masse- oder Volumenstrom der zugeführten Luft wird beim Strömen durch den Strömungstubus ebenfalls erfasst. Aus dem Zeitpunkt, zu dem auf der Ausgabeeinrichtung ein Ausgangssignal ausgegeben wird, kann erkannt werden, ob sich die Ausgabe auf einen Masse- oder Volumenstrom einer von dem Hilfeleistenden zugeführten Luft oder einer von der Person ausströmenden Luft bezieht. Wird zeitgleich zu der Zufuhr von Luft ein Ausgangssignal mit der Ausgabeeinrichtung ausgegeben, so bezieht sich dieses auf die zugeführte Luft. Wird dagegen ein Ausgangssignal ausgegeben, ohne dass Luft durch den Hilfeleistenden zugeführt wird oder zeitlich versetzt zur Luftzufuhr, so bezieht sich das Ausgangssignal auf eine von der Person ausströmende Luft.

Mit der Vorrichtung kann erfasst werden, ob bei dem Unfall- oder Notfallopfer die Atmung ganz oder teilweise ausgesetzt hat, bevor eine Beatmung oder eine Herzdruckmassage durchgeführt wird. Hat die Atmung der Person ausgesetzt, so wird mit dem Strömungssensor kein Masse- oder Volumenstrom nachgewiesen. Es erscheint somit kein entsprechendes Ausgangssignal. Wird dagegen ein Masse- oder Volumenstrom erfasst, so ist dies ein Zeichen dafür, dass bei der Person eine Spontanatmung stattfindet. Ferner kann festgestellt werden, ob die durch den Hilfeleistenden zugeführte Atemluft ausreicht um den Atemstillstand zu beenden.

Wird eine Herzdruckmassage durchgeführt, so wird der Brustkorb der Person durch Krafteinwirkung komprimiert. Nach Wegfall der Krafteinwirkung findet eine Dekomprimierung statt. Der Brustkorb dehnt sich wieder aus. Dieser Vorgang wird mehrfach wiederholt. Bei der Komprimierung des Brustkorbs werden insbesondere das Herz und die Lunge der Person zusammengedrückt. Hierdurch wird in einem eingeschränkten Umfang ein Blutkreislauf unterstützt, der die Versorgung lebenswichtiger Organe, insbesondere des Gehirns der Person ermöglicht. Die Komprimierung der Lunge führt dazu, dass aus Nase oder Mund der Person Luft ausströmt, sofern die Atemwege der Person nicht verlegt sind. Wird daher bei einer Herzdruckmassage während der Komprimierung des Brustkorbs der Person kein Masse- oder Volumenstrom der Person mittels des Strömungssensors nachgewiesen, so ist dies ein Zeichen dafür, dass die Atemwege der Person verlegt sind. Dies gilt insbesondere, wenn trotz einer Steigerung der Eindringtiefe bei der Herzdruckmassage kein Masse- oder Volumenstrom erfasst wird. Die Atemwege können durch die Zunge der Person oder durch Erbrochenes verlegt sein.

Sind die Atemwege frei, so kann anhand des mit dem Strömungssensors erfassten Masse- oder Volumenstroms festgestellt werden, ob die Eindringtiefe bei der Herzdruckmassage ausreicht, um eine für die Reanimation und/ oder für die Versorgung bestimmter Organe notwendiger Kreislauf aufrechterhalten werden kann. Hierfür sind bestimmte Bereiche vorgegeben. Die Eindringtiefe entspricht dabei der Strecke, um die der Brustkorb bei der Komprimierung eingedrückt wird. Der Masse- oder Volumenstrom, der bei der Komprimierung des Brustkorbs nachgewiesen wird, steht dabei in Relation zu der Eindringtiefe des Brustkorbs.

Ob die Atemwege der Person verlegt sind, kann auch festgestellt werden, in dem ein Hilfeleistender Luft über das Mundstück der Vorrichtung insuffliert und der Strömungssensor den Masse- oder Volumenstrom erfasst. Der Masse- oder Volumenstrom im Strömungstubus hängt davon ab, ob die Atemwege frei oder verlegt sind und ob gegebenenfalls sogar die Speiseröhre der Person blockiert ist. Sind die Atemwege der Person frei, gelangt die von dem Hilfeleistenden zugeführte Luft in die Lunge der Person. Sind die Atemwege verlegt und die Speiseröhre blockiert, so gelangt die von einem Hilfeleistenden zugeführte Luft in den Bauch der Person. Sind die Atemwege und die Speiseröhre blockiert so gelangt die von dem Hilfeleistenden zugeführte Luft nur in den Mund der Person. Die drei genannten Fälle weisen einen typischen Verlauf des Masse- oder Volumenstroms auf, der in der Vorrichtung als Kennlinie hinterlegt sein kann. Anhand des Vergleichs des zeitlichen Verlaufs des Masse- oder Volumenstroms mit diesen Kennlinien kann festgestellt werden, ob die Atemwege verlegt sind, auch dann wenn keine Herzdruckmassage durchgeführt wird. Hierzu genügt eine Beatmung durch Zufuhr von Luft über das Mundstück.

Die Vorrichtung ist mit einem Prozessor und einer Ausgabeeinrichtung ausgestattet. In dem Prozessor wird der mit dem Strömungssensor erfasste Masse- oder Volumenstrom verarbeitet. Hierzu werden die Signale des Strömungssensors aufbereitet. Es wird ein Ausgangssignal erzeugt, das mit der Ausgabeeinrichtung ausgegeben wird.

Der Prozessor ist als Eindringtiefe-Signal erzeugender Prozessor ausgebildet. Bei einer Herzdruckmassage wird der Brustkorb der in einem medizinischen Notfall befindlichen Person um eine Strecke zusammengedrückt, die als Eindringtiefe bezeichnet wird. Bei dieser Komprimierung des Brustkorbs strömt aus Nase und/ oder Mund der Person Luft aus. Sie gelangt über die Beatmungsmaske in den Strömungstubus und umströmt dort den Strömungssensor, anhand dessen der Masse- oder Volumenstrom der von der Person ausströmenden Luft erfasst wird. Der Masse- oder Volumenstrom steht in Relation zu der Eindringtiefe. Der Prozessor erzeugt aus dem erfassten Masse- oder Volumenstrom ein für die Eindringtiefe charakteristisches Eindringtiefe-Signal. Die Ausgabeeinrichtung ist ausgebildet, das Eindringtiefe-Signal als Ausgangssignal auszugeben. Das Eindringtiefe-Signal ist damit ein Ausgangssignal wie oben angegeben. Damit erhält der Hilfeleistende eine Information darüber, ob die Eindringtiefe, bis zu der er den Brustkorb der Person zusammengedrückt hat, ausreicht, um die Person bis zum Eintreffen professioneller Rettungskräfte zu versorgen.

Ein Hilfeleistender erhält somit eine Unterstützung bei der ersten Hilfe. Die erste Hilfe kann optimal an die Bedürfnisse der Person angepasst werden. Auf diese Weise können Hemmschwellen bei potentiellen Hilfeleistenden abgebaut werden.

Die Erfassung der Parameter der in dem Strömungstubus strömenden Gase dauert auch während der ersten Hilfe an. Dadurch können die an den Hilfeleistenden ausgegebenen Anweisungen bei fortschreitender Hilfe stets an den gegebenenfalls sich ändernden Zustand des Notfallopfers angepasst werden.

Die Vorrichtung ist bevorzugt mit einem Energiespeicher ausgestattet, beispielsweise eine Batterie.

Vorteilhafterweise weist die Vorrichtung eine Speichereinrichtung auf. In der Speichereinrichtung werden Daten, Signale und/ oder Messwerte betreffend den erfassten Masse- oder Volumenstrom oder daraus abgeleitete Größen abgespeichert. Auch Kennlinien betreffend den typischen zeitlichen Verlauf bei Zufuhr von Luft, wenn die Atemwege frei oder verlegt und gegebenenfalls auch die Speiseröhre blockiert ist, können in der Speichereinrichtung abgelegt sein. Darüber hinaus können in der Speichereinrichtung für verschiedene Gruppen von Personen, beispielsweise Erwachsene, Kinder und Kleinkinder, unterschiedliche Bereiche eines für die Reanimation ausreichenden Masse- oder Volumenstroms abgelegt sein. In der Speichereinrichtung können ferner zu den möglichen Messwerten oder Messwertbereichen Empfehlungen für das Vorgehen eines Hilfeleistenden abgespeichert sein. Der Prozessor ordnet in diesem Fall den mit dem Sensor erfassten Messwerten entsprechende im Speicher abgelegte Anweisungen oder Empfehlungen zu. Diese werden über eine Ausgabeeinrichtung an den Hilfeleistenden ausgegeben. Auf diese Weise kann der Hilfeleistende kontinuierlich darüber informiert werden, welche Schritte er vorzunehmen hat, wobei die Schritte auf den jeweiligen Zustand des Unfall- oder Notfallopfers abgestimmt sind. Der Hilfeleistende wird beispielsweise darüber informiert, wann er dem Unfall- oder Notfallopfer Atemluft zuführen soll, wann er mit der Herzdruckmassage beginnen soll, wie oft und in welchem zeitlichen Abstand er Druck auf den Brustbereich des Unfall- oder Notfallopfers ausüben soll, bevor er erneut Atemluft zuführt.

Nach einer vorteilhaften Ausgestaltung der Erfindung ist mindestens ein weiterer Sensor an dem Strömungstubus vorgesehen, der Parameter des Luftstroms und/ oder der in der strömenden Luft enthaltenen Gase erfasst. Die Parameter sind insbesondere physikalische oder chemische Eigenschaften der Gase. Hierzu zählen beispielsweise:
- der Atemwegsdruck relativ zur Umgebung,
- der Umgebungsdruck,
- die Druckdifferenz,
- die Temperatur,
- die Feuchtigkeit,
- der Sauerstoffgehalt oder die Sauerstoffkonzentration,
- der Kohlendioxidgehalt oder die Kohlendioxid-Konzentration.

Der oder die Sensoren sind ausgebildet, um die genannten Messgrößen zu erfassen. Die Parameter wie Atemströmung, Druckdifferenz, Sauerstoff- oder Kohlendioxid-Konzentration werden auch als Vitalwerte der Person bezeichnet. Sensoren, welche die genannten Parameter erfassen, werden daher auch als Vitalwert-Sensoren bezeichnet.

Nach einer vorteilhaften Ausgestaltung der Erfindung ist die Vorrichtung zusätzlich zu dem Strömungssensor mit einem Vitalwert-Sensor ausgestattet ist, mittels dem ein Anteil an Sauerstoff und Kohlendioxid in der durch den Strömungskanal hindurchströmenden Luft erfassbar ist. Der Prozessor ist derart ausgebildet, dass er den erfassten Anteil an Sauerstoff und Kohlendioxid zu einem Ausgangssignal verarbeitet, welches mit der Ausgabeeinrichtung ausgegeben wird.

In der Lunge der Person wird Sauerstoff in Kohlendioxid umgewandelt. Diese Umwandlung findet auch bei einer Beatmung oder einer Herzdruckmassage statt, wenn die Person nicht selbsttätig atmet und ein Kreislaufstillstand vorliegt. Wird daher mit dem Sensor nachgewiesen, dass bei der aus der Person ausströmenden Luft die Kohlendioxid-Konzentration größer ist, als bei der zugeführten Luft, so ist dies ein Anzeichen dafür, dass die aufgrund von Beatmung oder Herzdruckmassage zugeführte Luft in die Lunge der Person gelangt ist. Auch ein entsprechender Nachweis eines Kohlendioxidgehaltes oder einer Kohlendioxidkonzentration kann somit Aufschluss über einer Beatmung oder Herzdruckmassage geben, insbesondere ob die jeweilige Maßnahme die Anforderung an eine Reanimation erfüllt.

Nach einer vorteilhaften Ausgestaltung der Erfindung wird mittels des erfassten Anteils an Sauerstoff und Kohlendioxid die Eindringtiefe bei einer Herzdruckmassage bestimmt. Diese hängt von dem erfassten Anteil an Sauerstoff und Kohlendioxid ab.

Nach einer vorteilhaften Ausgestaltung der Erfindung ist die Ausgabeeinrichtung eine optische Anzeigeeinrichtung. Die optische Anzeigeeinrichtung weist beispielsweise einen Bildschirm oder einen Monitor auf.

Nach einer weiteren vorteilhaften Ausgestaltung der Erfindung weist die optische Anzeigeeinrichtung eine Kombination mehrerer Lichtquellen auf. Als Lichtquellen eignen sich beispielsweise Licht emittierende Dioden in unterschiedlichen Farben. Die unterschiedlichen Farben können für verschiedene Bereiche des mit dem Strömungssensor gemessenen Masse- oder Volumenstroms der Person oder einer daraus abgeleiteten Größe stehen. Zum Beispiel kann eine erste Farbe für einen Masse- oder Volumenstrom stehen, der kleiner oder gleich ist wie ein vorgegebenes Minimum. Eine zweite Farbe kann für einen Masse- oder Volumenstrom stehen, der größer oder gleich ist wie ein vorgegebenes Maximum. Eine dritte Farbe kann für einen Masse- oder Volumenstrom stehen, der zwischen dem Minimum und dem Maximum liegt. Dabei können für verschiedene Personengruppen, beispielsweise Erwachsene, Kinder und Kleinkinder, unterschiedliche Minima und Maxima vorgegeben sein. Diese sind bevorzugt in einer Speichereinrichtung der Vorrichtung abgelegt. Die Vorrichtung ist in diesem Fall vorteilhafterweise mit einer Eingabeeinrichtung ausgestattet, über die die Personengruppe eingegeben werden, der die in einem Notfall befindliche Person angehört.

Nach einer weiteren vorteilhaften Ausgestaltung der Erfindung weist die optische Anzeigeeinrichtung eine optisch aktive Oberfläche auf, an der das Ausgangssignal angezeigt wird. Diese optisch aktive Oberfläche ist vorteilhafterweise nach außen gewölbt.

Alternativ dazu kann die Oberfläche auch im wesentlichen als ebene Oberfläche ausgebildet sein. Vorteilhafterweise ist die ebene optisch aktive Oberfläche unter einem Winkel von mehr als 0° und weniger als 90° gegen die Längsrichtung des Strömungstubus ausgerichtet. Dabei entspricht die Längsrichtung des Strömungstubus im wesentlichen der Strömungsrichtung der durch den Strömungstubus strömenden Gase. Die Wölbung der optisch aktiven Oberfläche oder die Neigung der optisch aktiven Oberfläche gegen den Strömungstubus erleichtert dem Hilfeleistenden das Erkennen des auf der Anzeigeeinrichtung angezeigten Ausgangssignals, während sich die Vorrichtung an der von einem Notfall betroffenen Person befindet.

Nach einer weiteren vorteilhaften Ausgestaltung der Erfindung weist die Ausgabeeinrichtung eine akustische Ausgabeeinrichtung auf. In diesem Fall wird das Ausgangssignal beispielsweise durch ein Mikrophon an den Hilfeleistenden ausgegeben. Optische Anzeigeeinrichtung und akustische Ausgabeeinrichtung können auch miteinander kombiniert sein.

Nach einer weiteren vorteilhaften Ausgestaltung der Erfindung umfasst die Ausgabeeinrichtung eine Schnittstelle zur Ausgabe von Daten betreffend einen durch den Strömungssensor erfassten Masse- oder Volumenstrom oder daraus abgeleitete Daten an ein mobiles Telekommunikationsgerät. Es kann sich um eine Schnittstelle zur Datenübertragung per Funk, wie beispielsweise Bluetooth, oder um eine Schnittstelle zur Datenübertragung über eine Datenleitung handeln. Das Ausgangssignal kann in diesem Fall auch über das mobile Telekommunikationsgerät ausgegeben werden. Darüber hinaus können in einer Speichereinrichtung des Telekommunikationsgerätes die Ausgangssignale abgespeichert werden, so dass sie zu einem späteren Zeitpunkt abrufbar sind.

Es können auch mehrere verschiedene Ausgabeeinrichtungen vorgesehen sein, so dass die zu unternehmenden Schritte auf unterschiedliche Art an den Hilfeleistenden ausgegeben werden.

Nach einer weiteren vorteilhaften Ausgestaltung ist die Vorrichtung mit einer Schnittstelle ausgestattet, über die Daten betreffend einen durch den Strömungssensor erfassten Masse- oder Volumenstrom oder daraus abgeleitete Daten an ein externes Gerät zur Notfallversorgung, an ein medizinisches Gerät oder an einen Computer ausgebbar sind. Die Daten sind damit abrufbar und stehen im weiteren Verlauf der Versorgung des Patienten zur Verfügung. Ein Rettungssanitäter, ein Arzt oder eine Pflegekraft können sich somit schnell und einfach einen Eindruck über den Zustand der Peron und die bereits geleistet erste Hilfe verschaffen. Bei dem medizinischen Gerät kann es sich beispielsweise um ein Beatmungsgerät oder um einen Defibrillator handeln.

Nach einer weiteren vorteilhaften Ausgestaltung der Erfindung ist in dem Strömungskanal zwischen dem Mundstück und dem Strömungssensor ein erstes Filter angeordnet. Das Filter sorgt dafür, dass der Strömungssensor möglichst gleichmäßig von der durch das Filter einströmenden Luft umströmt wird. Ziel ist es, eine möglichst laminare Strömung zu erzeugen. Darüber hinaus verhindert das Filter, dass Teile in den Strömungstubus gelangen und den Strömungssensor beschädigen können. Das erste Filter kann beispielsweise aus einem Gitter oder Geflecht aus Kunststoff oder Metall, insbesondere Draht, aus einem Vliesmaterial oder einem Gewirk aus Metall und/ oder Kunststoff bestehen.

Nach einer weiteren vorteilhaften Ausgestaltung der Erfindung ist in dem Strömungskanal zwischen der Beatmungsmaske und dem Strömungssensor ein zweites Filter angeordnet. Genau wie das erste Filter begünstigt es die Strömung der Luft oder des Luftgemischs durch den Strömungstubus entlang des Sensors mit dem Ziel, eine möglichst laminare Strömung zu erzeugen. Ferner verhindert das zweite Filter das Endringen von Teilen in den Strömungstubus, die den Strömungssensor beschädigen könnten. Hierzu zählen auch Absonderungen der in einem medizinischen Notfall befindlichen Person. Das zweite Filter kann beispielsweise aus einem Gitter oder Geflecht aus Kunststoff oder Metall, insbesondere Draht, aus einem Vliesmaterial oder einem Gewirk aus Metall und/ oder Kunststoff bestehen.

Nach einer weiteren vorteilhaften Ausgestaltung der Erfindung ist ein drittes Filter zwischen der Beatmungsmaske und dem Strömungssensor angeordnet, welches die Feuchtigkeit in der aus der Person ausströmenden Luft zurück hält und dadurch verhindert, dass die Lunge der Person zu sehr austrocknet. Dieses dritte Filter kann zusätzlich Absonderungen der Person zurück halten. Das dritte Filter kann beispielsweise aus einem Gitter oder Geflecht aus Kunststoff oder Metall, insbesondere Draht, aus einem Vliesmaterial oder einem Gewirk aus Metall und/ oder Kunststoff bestehen.

Nach einer weiteren vorteilhaften Ausgestaltung der Erfindung ist der Strömungstubus hinsichtlich des Querschnitts an seinem dem Mundstück zugewandten Ende an bekannte Beatmungsgeräte und deren Zubehör angepasst. Beim Eintreffen von professionellen Rettungskräften können diese das Mundstück von dem Strömungstubus abnehmen und ein Beatmungsgerät an den Strömungstubus anschließen. Die Beatmungsmaske und der Strömungstubus können am Patienten verbleiben.

Nach einer weiteren vorteilhaften Ausgestaltung der Erfindung ist an einer Wand des Strömungstubus eine Sensorhalteeinrichtung angeordnet. Die Sensorhalteeinrichtung ragt zumindest abschnittsweise in den Strömungskanal des Strömungstubus hinein. An dem in den Strömungskanal hineinragenden Abschnitt der Sensorhalteeinrichtung ist der Strömungssensor angeordnet. Auf diese Weise ist der Strömungssensor derart an dem Strömungstubus gehalten, dass er in das im Strömungstubus strömende Gas hineinragt und den Masse- oder Volumenstrom zuverlässig erfasst.

Nach einer weiteren vorteilhaften Ausgestaltung der Erfindung ist die Vorrichtung mit einem Aufsteckteil ausgestattet, in welchem der Prozessor und die Ausgabeeinrichtung angeordnet sind. Optional sind zusätzlich eine Speichereinrichtung und gegebenenfalls eine oder mehrere Schnittstellen angeordnet in dem Aufsteckteil angeordnet. Das Aufsteckteil ist lösbar mit dem Strömungstubus verbunden. Es kann vom Strömungstubus gelöst werden, ohne dass das Aufsteckteil oder der Strömungstubus dabei beschädigt oder zerstört werden. Das Aufsteckteil weist ein Gehäuse auf, in dem der Prozessor und die Ausgabeeinrichtung gut geschützt angeordnet sind. Da es nicht mit dem Patienten und der Atemluft in Berührung kommt, kann das Aufsteckteil wiederverwendet werden. Der Strömungstubus, das Mundstück und die Beatmungsmaske können nach Gebrauch entweder entsorgt werden oder gereinigt, desinfiziert und wiederverwendet werden.

Nach einer weiteren vorteilhaften Ausgestaltung der Erfindung umgreift das Aufsteckteil den Strömungstubus zumindest teilweise.

Nach einer weiteren vorteilhaften Ausgestaltung der Erfindung weist das Aufsteckteil eine Armbefestigungseinrichtung auf, mit der es an einem Arm eines Hilfeleistenden befestigbar ist. Ferner ist das Aufsteckteil mit einem Beschleunigungssensor ausgestattet, welcher eine Beschleunigung des Arms bei einer Herzdruckmassage erfasst. Sollte mittels des Strömungssensors erfasst werden, dass die Atemwege der Person verlegt sind, und sollte es nicht möglich sein, die Atemwege frei zu räumen, so kann der Hilfeleistende das Aufsteckteil vom Strömungstubus lösen und an seinem Arm befestigen. Bei der Durchführung der Herzdruckmassage wird die Eindringtiefe nun nicht mehr über den erfassten Masse- oder Volumenstrom bestimmt sondern über die Beschleunigung, mit der der Hilfeleistende bei der Herzdruckmassage seinen Arm bewegt. Das Umschalten von dem Strömungssensor auf den Beschleunigungssensor erfolgt bevorzugt automatisch, wenn das Aufsteckteil von dem Strömungstubus gelöst wird. Dabei verbleibt der Strömungssensor an dem Strömungstubus.

Nach einer weiteren vorteilhaften Ausgestaltung der Erfindung ist der Prozessor derart ausgebildet, dass er aus der mit dem Beschleunigungssensor erfassten Beschleunigung ein Ausgangssignal erzeugt, welches mit der Ausgabeeinrichtung ausgebbar ist.

Nach einer weiteren vorteilhaften Ausgestaltung der Erfindung sind an dem Aufsteckteil mindestens zwei Griffmulden und/ oder nach außen abstehende Griffelemente angeordnet.

Nach einer weiteren vorteilhaften Ausgestaltung der Erfindung weist die Vorrichtung einen Vitalwert-Sensor auf. Mit diesem werden als Vitalwert einer Person zusätzlich zu dem Masse- oder Volumenstrom der durch den Strömungskanal strömenden Gase die Druckdifferenz in dem Strömungskanal und/ oder die Sauerstoff-Konzentration und/ oder die Kohlendioxid-Konzentration und/ oder andere Strömungsparameter bestimmt.

Nach einer weiteren vorteilhaften Ausgestaltung der Erfindung ist der Sensor als Atmungsantwort-Sensor ausgebildet, der die Atmungsantwortcharakteristik der Person erfasst, dass der Prozessor als Atmungsantwort-Prozessor ausgebildet ist, das die Atmungscharakteristik auswertet und feststellt, ob die Atemwege der Person verlegt sind. Über die Ausgabeeinrichtung wird das Ergebnis der Auswertung ausgegeben. Ein Hilfeleistender erhält auf diese Weise eine Information darüber, ob die Atemwege der zur beatmenden Person verlegt oder frei sind.

Nach einer weiteren vorteilhaften Ausgestaltung der Erfindung weist die Vorrichtung eine Eingabeeinrichtung auf, über die bestimmte Eigenschaften der Person eingebbar sind, die sich in einem medizinischen Notfall befindet. So kann ein Hilfeleistender beispielsweise anhand der Eingabeeinrichtung auswählen, ob es sich bei der Person um einen Erwachsenen, ein Kind oder ein Kleinkind handelt. Bei diesen Personengruppen sind die für eine Reanimation vorgegebenen Bereiche unterschiedlich, so dass der Hilfeleistende entsprechend informiert werden kann.

Das beschriebene Verfahren zeichnet sich dadurch aus, dass mit Hilfe der Vorrichtung nach einem der Ansprüche 1 bis 13 nach dem Aufsetzen der Beatmungsmaske auf eine von einem medizinischen Notfall betroffene Person die von der Person ausströmenden Gase, welche den Strömungstubus passieren, von einem in dem Strömungstubus angeordneten Strömungssensor erfasst werden. Hierzu wird der Masse- oder Volumenstrom des in dem Strömungstubus strömenden Gases erfasst. Der erfasste Masse- oder Volumenstrom wird mit Hilfe des Prozessors zu einem Ausgangssignal verarbeitet. Das Ausgangssignal wird mit der Ausgabeeinrichtung ausgegeben.

Der Hilfeleistende erhält vorteilhafterweise nicht nur allgemeine Hinweise für die Atemspende oder die Herzdruckmassage sondern konkrete Anweisungen, die an den Zustand des Notfallopfers angepasst sind. Die Erfassung des Masse- oder Volumenstroms und die Ausgabe des Ausgangssignals dauert während der ersten Hilfe an. Dadurch können die an den Hilfeleistenden ausgegebenen Anweisungen bei fortschreitender Hilfe stets an den gegebenenfalls sich ändernden Zustand des Notfallopfers angepasst werden.

Nach einer weiteren vorteilhaften Ausgestaltung der Erfindung werden von dem Strömungssensor die in beide Richtungen in dem Strömungstubus strömenden Gase erfasst. Es erfolgt damit nicht nur eine Erfassung des Masse- oder Volumenstroms der von der Person ausströmenden Luft sondern auch der dem Patienten zugeführten Luft.

Nach einer weiteren vorteilhaften Ausgestaltung des beschriebenen Verfahrens wird mit dem Strömungssensor ein Masse- oder Volumenstrom der bei einer Herzdruckmassage aufgrund der Komprimierung des Brustkorbs bis zu einer Eindringtiefe aus Nase und/ oder Mund der Person ausströmenden und durch den Strömungstubus hindurchströmenden Luft erfasst. Mit dem Prozessor wird ein für die Eindringtiefe charakteristisches Eindringtiefe-Signal aus dem erfassten Masse- oder Volumenstrom erzeugt. Das Eindringtiefe-Signal wird als Ausgangssignal mit der Ausgabeeinrichtung ausgegeben.

Nach einer weiteren vorteilhaften Ausgestaltung des beschriebenen Verfahrens wird mit der Ausgabeeinrichtung angezeigt, ob der erfasste Masse- oder Volumenstrom oder das daraus abgeleitete Eindringtiefe-Signal oder eine sonstige aus dem Masse- oder Volumenstrom abgeleitete Größe innerhalb eines für eine Reanimation vorgegebenen Bereichs liegt. Der Bereich wird derart vorgegeben, dass in diesem Bereich ein Blutkreislauf stattfindet, der für eine Durchblutung des Gehirns sorgt.

Nach einer weiteren vorteilhaften Ausgestaltung des beschriebenen Verfahrens wird vor Beginn einer Herzdruckmassage der Masse- oder Volumenstrom der aus Nase und/ oder Mund der Person ausströmenden Luft mittels des Strömungssensors erfass. Mittels der Ausgabeeinrichtung wird ausgegeben, ob der erfasste Masse- oder Volumenstrom innerhalb eines für eine Spontanatmung vorgegebenen Bereichs liegt. Auf diese Weise kann festgestellt werden, ob die Person selbständig atmet.

Nach einer weiteren vorteilhaften Ausgestaltung der Erfindung wird zusätzlich während der Komprimierung des Brustkorbs mittels eines Beschleunigungssensors die Beschleunigung erfasst, mit der der Brustkorb komprimiert wird. Aus der erfassten Beschleunigung wird ein für die Eindringtiefe charakteristisches Eindringtiefe-Signal erzeugt. Das Eindringtiefe-Signal wird mittels der Ausgabeeinrichtung als Ausgangssignal ausgegeben. Mittels der Ausgabeeinrichtung wird angezeigt, ob die erfasste Beschleunigung oder das daraus abgeleitete Eindringtiefe-Signal oder eine sonstige aus der Beschleunigung abgeleitete Größe innerhalb eines für eine Reanimation vorgegebenen Bereichs liegt.

Nach einer weiteren vorteilhaften Ausgestaltung der Erfindung wird das Ausgangssignal nicht nur an den Hilfeleistenden ausgegeben sondern an Dritte, die an der späteren medizinischen Versorgung des Notfallopfers beteiligt sind. Hierbei handelt es sich typischer Weise nicht um Ersthelfer sondern um medizinische Fachleute wie Sanitäter, Pfleger oder Ärzte.

Weitere Vorteile und vorteilhafte Ausgestaltungen der Erfindung sind der nachfolgenden Beschreibung, der Zeichnung und den Ansprüchen entnehmbar.

### Zeichnung

In der Zeichnung sind Ausführungsbeispiele der Erfindung dargestellt. Es zeigen:
- Figur 1: erstes Ausführungsbeispiel einer Vorrichtung zur Atemspende in Notfällen in Seitenansicht,
- Figur 2: Vorrichtung gemäß Figur 1 in ihre einzelnen Komponenten zerlegt,
- Figur 3: Strömungstubus mit Aufsteckteil der Vorrichtung gemäß Figur 1 in perspektivischer Ansicht,
- Figur 4: Strömungstubus mit Aufsteckteil gemäß Figur 3 in einer Ansicht von unten,
- Figur 5: Strömungstubus gemäß Figur 3 mit abgehobenem Aufsteckteil,
- Figur 6: Strömungstubus gemäß Figur 3 in Explosionsdarstellung,
- Figur 7: Ausschnitt aus Figur 6 betreffend den Sensor, die Sensorleiterplatte und die Sensorhülsen
- Figur 8: Aufsteckteil gemäß Figur 3
- Figur 9: zweites Ausführungsbeispiel einer Vorrichtung zur Atemspende in Notfällen in einer Ansicht von vorne,
- Figur 10: Vorrichtung gemäß Figur 9 in Seitenansicht,
- Figur 11: Strömungstubus und Aufsteckteil der Vorrichtung gemäß Figur 9 in perspektivischer Ansicht,
- Figur 12: Strömungstubus der Vorrichtung gemäß Figur 9 in perspektivischer Ansicht,
- Figur 13: Aufsteckteil der Vorrichtung gemäß Figur 9 in perspektivischer Ansicht von oben,
- Figur 14: Aufsteckteil der Vorrichtung gemäß Figur 9 in perspektivischer Ansicht von unten,
- Figur 15: Aufsteckteil der Vorrichtung gemäß Figur 9 am Arm eines Hilfeleistenden angeordnet.

### Beschreibung der Ausführungsbeispiele

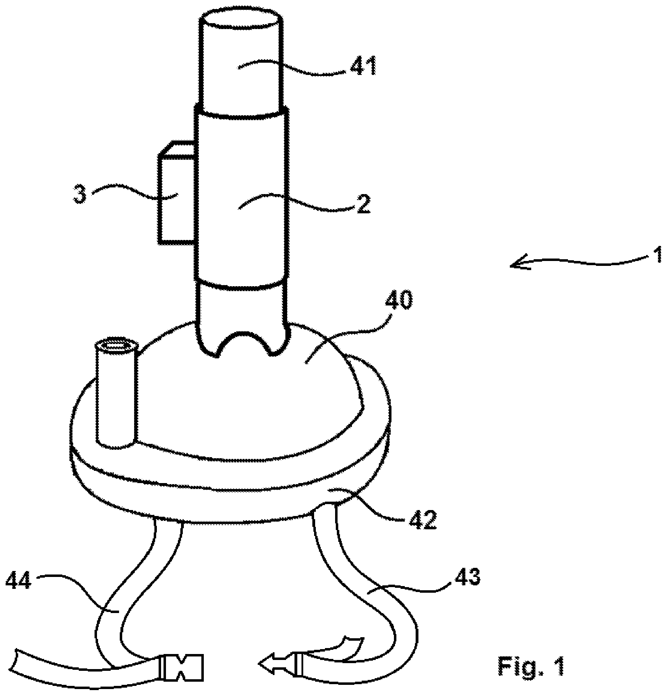

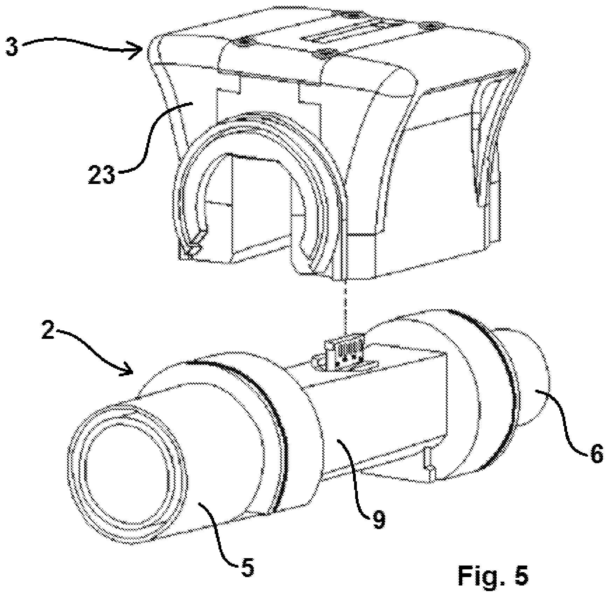

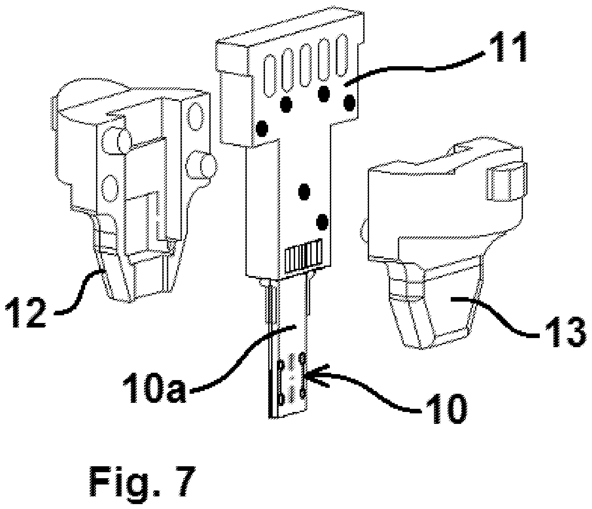

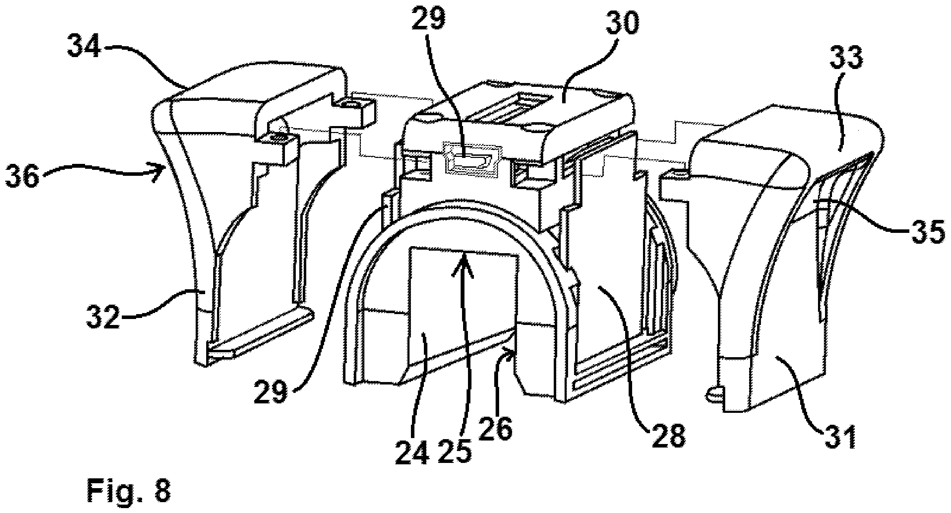

In den Figuren 1 bis 8 ist ein erstes Ausführungsbeispiel einer Vorrichtung zur Atemspende in Notfällen dargestellt. Die Vorrichtung weist eine Beatmungsmaske 40, ein Mundstück 41, einen Strömungstubus 2 und ein Aufsteckteil 3 auf. Die Beatmungsmaske ist mit einem elastischen Rand 42 ausgestattet, damit sie auf dem Gesicht eines Unfall- oder Notfallopfers luftdicht abschließt. Die Beatmungsmaske 40 wird üblicherweise mit einem gewissen Druck auf das Gesicht einer Person gepresst, damit am Rand keine Luft entweichen kann. Zum Befestigen der Beatmungsmaske an einer Person ist an zwei Seiten der Beatmungsmaske jeweils ein Band 43, 44 angeordnet. Die beiden Bänder 43, 44 werden um den Kopf einer Person gelegt und miteinander verbunden. Dadurch wird die Beatmungsmaske und mit ihr die gesamte Vorrichtung an einer Person fixiert.

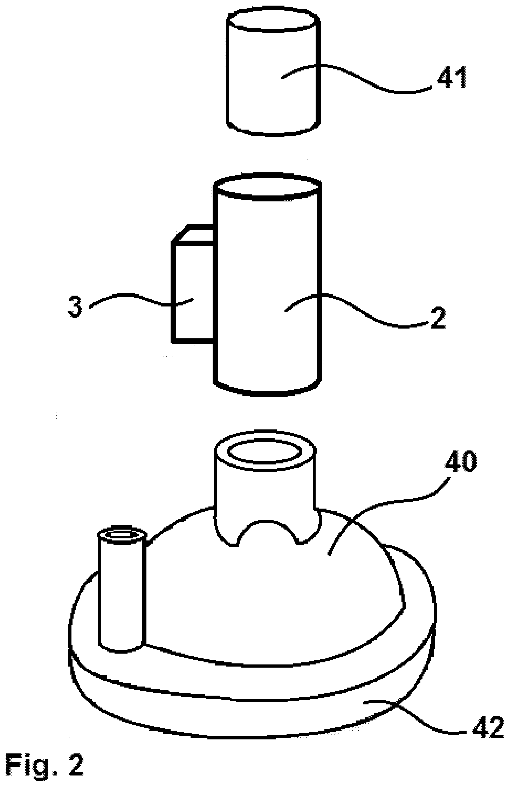

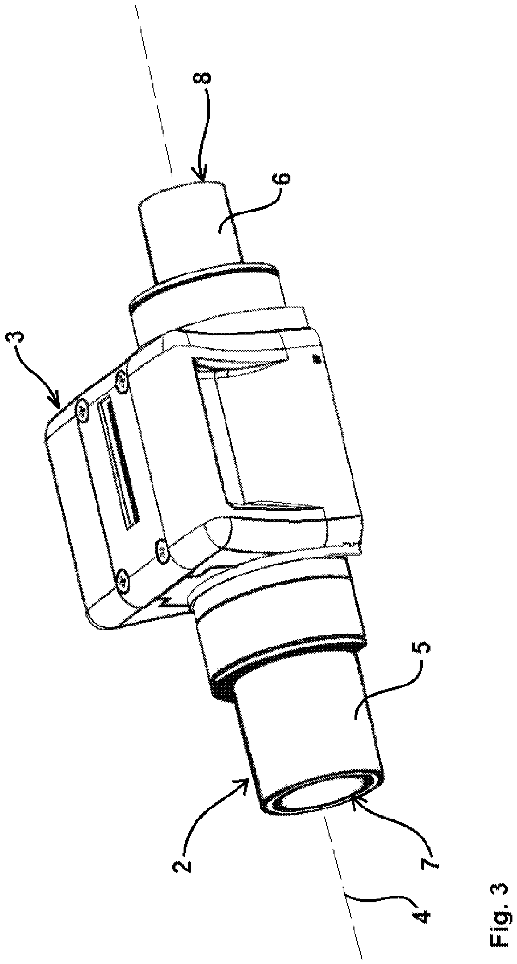

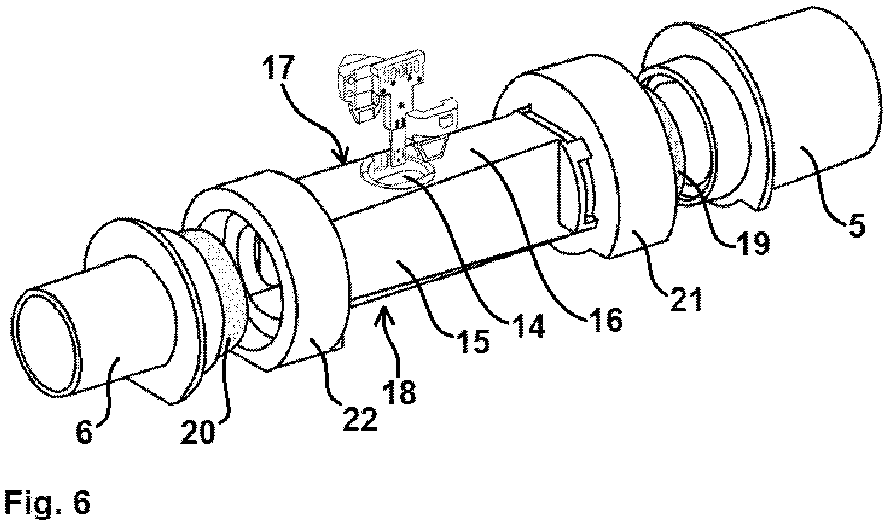

In den Figuren 3 bis 8 ist der in den Figuren 1 und 2 gezeigte Strömungstubus 2 mit dem Aufsteckteil 3 dargestellt. Der Strömungstubus ist ein länglicher Hohlkörper. Seine Längsachse 4 entspricht der Strömungsrichtung der durch den Strömungstubus strömenden Luft. Der Strömungstubus 2 weist an seinen beiden Enden je einen zylindrischen Endabschnitt 5, 6 mit kreisrunder Querschnittsfläche auf. Mit diesen Endabschnitten 5, 6 wird der Strömungstubus 2 in die Beatmungsmaske 40 einenends und in das Mundstück 41 anderenends eingeführt. An der ersten Stirnseite 7 und an der zweiten Stirnseite 8 ist der Strömungstubus 2 offen. Darüber hinaus ist der Strömungstubus im wesentlichen geschlossen. Zwischen den Endabschnitten 5, 6 mit dem kreisrunden Querschnitt befindet sich ein Sensorabschnitt 9 des Strömungstubus 2. An dem Sensorabschnitt 9 ist ein Strömungssensor 10 angeordnet. Die Befestigung des Sensors 10 ist in Figur 6 erkennbar. Der Strömungssensor 10 ist mit einer Sensorhalteeinrichtung 10a und einer Sensorleiterplatte 11 ausgestattet. An der Sensorhalteeinrichtung 10a können neben dem Strömungssensor 10 auch weitere Sensoren angeordnet werden. Die Sensorleiterplatte 11 bildet eine Verlängerung der Sensorhalteeinrichtung 10a. Die Sensorhalteeinrichtung 10a kann auch Teil der Sensorleiterplatte 11 sein. Die Sensorleiterplatte 11 wird von zwei Sensorhülsen 12 und 13 gehalten. Die Sensorhülsen sind an eine Sensoröffnung 14 im Sensorabschnitt 9 angepasst. Mit Hilfe der Sensorhülsen 12, 13 wird die Sensorleiterplatte 11 in die Sensoröffnung 14 gepresst und der Strömungssensor damit fixiert. Dabei wird die Sensorleiterplatte 11 zwischen den beiden Sensorhülsen 12, 13 eingeklemmt und die Sensoröffnung 14 verschlossen. Der Sensor ragt dann in den Strömungskanal des Strömungstubus 2 hinein.

Der Sensorabschnitt 9 wird durch vier Seitenwände 15, 16, 17, 18 begrenzt. Die beiden Seitenwände 15 und 17 sind eben und verlaufen unter einem Winkel von 5° zueinander. Der Winkel kann auch zwischen 2° und 20° betragen. Die Seitenwand 16 ist ebenfalls eben. Sie ist benachbart zu den beiden Seitenwänden 15 und 17. Die vierte Seitenwand 18 kann ebenfalls eben oder auch nach außen gewölbt sein. Ist die vierte Seitenwand 18 eben ausgebildet, so ist die bevorzugt parallel zu der Seitenwand 16. Die vier Seitewände 15, 16, 17, 18 erzeugen die Form eines Pyramidenstumpfs. Der Querschnitt des Sensorabschnitts 9 ist somit an dem dem zylindrischen Endabschnitt 5 des Strömungstubus 2 zugewandten Ende kleiner als an dem dem zylindrischen Endabschnitt 6 des Strömungstubus 2 zugewandten Ende. Die ist besonders gut in Figur 2 erkennbar. Zwischen den beiden Enden verkleinert sich der Querschnitt senkrecht zur Längsachse des Strömungstubus 2 kontinuierlich. In der entgegengesetzten Richtung vergrößert sich der Querschnitt kontinuierlich.

In Figur 6 ist sind ein erstes Filter 19 und ein zweites Filter 20 erkennbar. Sie sind an den dem Sensorabschnitt 9 zugewandten Enden der beiden Endabschnitte 5, 6 des Strömungstubus 2 angeordnet. Der Sensorabschnitt 9 ist an seinen beiden Enden mit Muffen 21, 22 ausgestattet, die die zylindrischen Endabschnitte 5, 6 umgreifen.

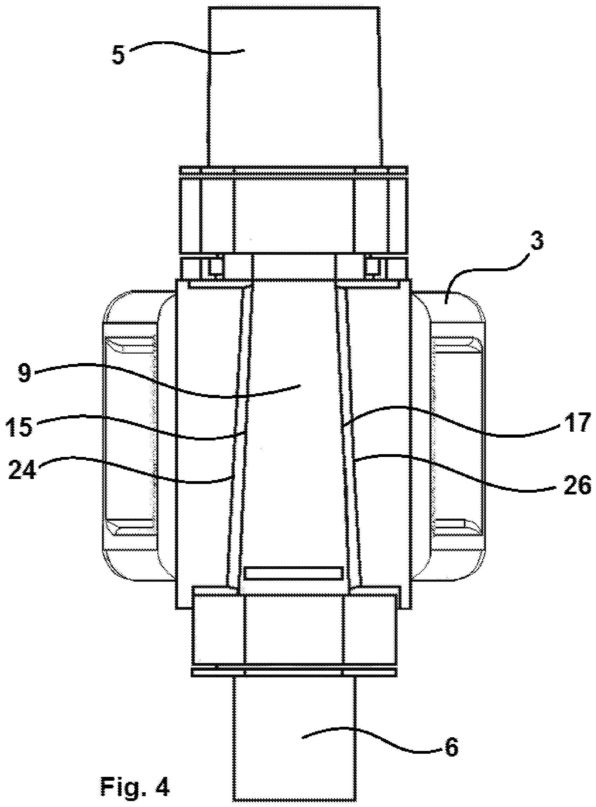

Das Aufsteckteil 3 weist ein Gehäuse 23 auf. Das Gehäuse 23 ist U-förmig. Es umgreift den Sensorabschnitt 9 des Strömungstubus 2 von drei Seiten, so dass die ebenen Seitenwände 15, 16 und 17 durch das Aufsteckteil vollständig abgedeckt sind. Hierzu weist das Aufsteckteil drei entsprechende ebene Seitenwände 24, 25 und 26 auf, die an den Seitenwänden 15, 16 und 17 anliegen. Dies ist im Bezug auf die Seitenwände 24 und 26 des Aufsteckteils 3 und im Bezug auf die Seitenwände 15 und 17 des Sensorabschnitts 9 in Figur 4 erkennbar. Durch die beiden einander gegenüber liegenden Seitenwände 24 und 26 des Aufsteckteils 3 wird eine Klemmkraft erzeugt, mit der das Aufsteckteil 3 an den Strömungstubus 2 beim Aufstecken gekoppelt wird.

In dem Gehäuse 23 des Aufsteckteils 3 sind Leiterplatten 27 und 28 angeordnet, an welchen elektrische und elektronische Komponenten angeordnet sind. Dies gilt insbesondere für einen Speicher und einen Prozessor. Darüber hinaus können ein Analog-Digital-Wandler und eine Gleichspannungsquelle an den Leiterplatten angeordnet sein. An der Seitenwand 25 ist eine in der Zeichnung nicht erkennbare Steckerbuchse angeordnet, in welche das obere Ende der Leiterplatte 11 des Sensors 10 eingesteckt wird. Die Steckerbuchse ist mit den Leiterplatten 27, 28 verbunden. Sie bildet die Schnittstelle zu dem Sensor 10. An der Vorderseite des Gehäuses 23 ist darüber hinaus eine Steckerbuchse 29 angeordnet, welche eine Schnittstelle zu einem externen, in der Zeichnung nicht dargestellten Lesegerät bildet. In einen Deckel 30 des Gehäuses 23 kann darüber hinaus eine Anzeigeeinrichtung integriert sein.

Die beiden äußeren Gehäuseteile 31 und 32 decken die Leiterplatten 27, 28 ab. Sie weisen ferner seitlich nach außen überstehende Griffelemente 33, 34 auf. Diese sind an ihrer Unterseite mit Griffmulden 35, 36 ausgestattet. Die Griffelemente 33, 34 und die Griffmulden 35, 36 erleichtern das Aufstecken des Aufsteckteils 3 auf den Strömungstubus 2 und das Abheben des Aufsteckteils 3 von dem Strömungstubus 2.

Zur Atemspende bei einem Notfallopfer wird die Beatmungsmaske 40 auf Nase und Mund des Notfallopfers ausgesetzt und an dem Kopf des Notfallopfers mit den Bändern 43, 44 befestigt. Der Sensor ist entweder bereits aktiviert oder wird gezielt aktiviert. Er erfasst die durch den Strömungstubus 2 strömenden Gase. Diese werden durch den Prozessor zu Messwerten verarbeitet. Der Prozessor ordnet den erfassten Messwerten eine zugehörige Anweisung zu, die in der Speichereinrichtung abgelegt ist. Diese wird auf der Anzeigeeinrichtung angezeigt und an den Hilfeleistenden ausgegeben. Die Erfassung der Parameter der in dem Strömungstubus strömenden Gase dauert auch während der ersten Hilfe an. Dadurch können die an den Hilfeleistenden ausgegebenen Anweisungen bei fortschreitender Hilfe stets an den gegebenenfalls sich ändernden Zustand des Notfallopfers angepasst werden.

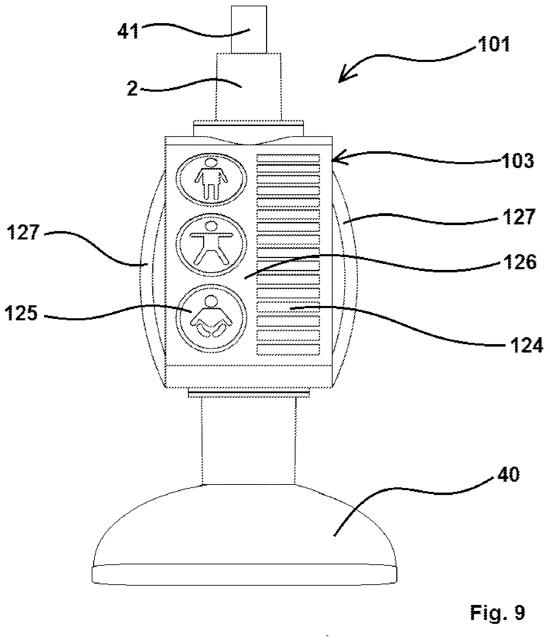

In den Figuren 9 bis 15 ist ein zweites Ausführungsbeispiel einer Vorrichtung 101 zur Atemspende in Notfällen dargestellt. Die Vorrichtung stimmt hinsichtlich der Beatmungsmaske 40, Mundstück 41 und Strömungstubus 2 im wesentlichen mit dem ersten Ausführungsbeispiel überein. Daher werden diesen Komponenten die gleichen Bezugszahlen zugeordnet. Das zweite Ausführungsbeispiel unterscheidet sich hinsichtlich des Aufsteckteils 103 von dem ersten Ausführungsbeispiel. In einem Gehäuse 123 des Aufsteckteils 103 sind ein in der Zeichnung nicht erkennbarer Prozessor und eine Ausgabeeinrichtung 124 angeordnet. Es handelt sich um eine optische Anzeigeeinrichtung mit mehreren übereinander parallel angeordneten länglichen Lichtelementen. Die einzelnen Lichtelemente weisen Licht emittierende Dioden in unterschiedlichen Farben auf. Ist nur das unterste Lichtelement eingeschaltet, so wird kein Masse- oder Volumenstrom im Strömungstubus erfasst. Ist nur das oberste Lichtelement eingeschaltet, so wird ein maximaler Volumenstrom erfasst. Oberstes und unterstes Lichtelement sind unterschiedlich in der Farbe. Die Lichtelemente dazwischen weisen eine dritte Farbe auf. Sie zeigen einen Masse- oder Volumenstrom zwischen dem Minimum und dem Maximum an. Die Symbole links neben den Lichtelementen sind auf Tasten oder Druckknöpfen angeordnet. Sie sind Teil einer Eingabeeinrichtung 125. Die Symbole stehen für Erwachsener, Kind oder Kleinkind. Durch Betätigen der jeweiligen Taste gibt der Hilfeleistende ein, welcher dieser drei Personengruppen die in einem medizinischen Notfall befindliche Person angehört. Das Maximum und Minimum des Masse- oder Volumenstroms der aus der Person ausströmenden Luft und die Eindringtiefe bei der Herzdruckmassage sind bei diesen drei Personengruppen unterschiedlich.

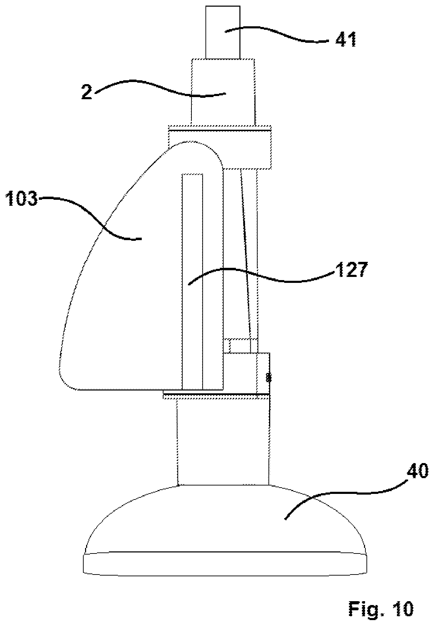

Die dem Betrachter in Figur 9 zugewandte Oberfläche des Aufsteckteils 103 ist eine optisch aktive Oberfläche 126. In diese Oberfläche 126 sind die Lichtelemente der Ausgabeeinrichtung 124 integriert. Diese Oberfläche ist nach außen gewölbt. Dies ist in der Seitenansicht gemäß Figur 10 erkennbar.

Das Aufsteckteil 103 weist seitlich an dem Gehäuse 123 zwei Griffelemente 127 auf. Mit Hilfe dieser Griffelemente kann das Aufsteckteil 103 von dem Strömungstubus 2 abgenommen werden.

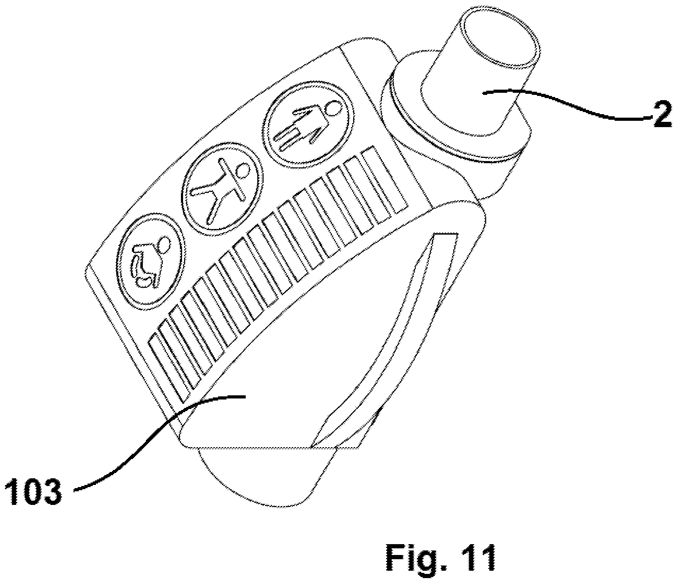

Figur 11 zeigt den Strömungstubus 2 und das Aufsteckteil 103 in einer perspektivischen Ansicht.

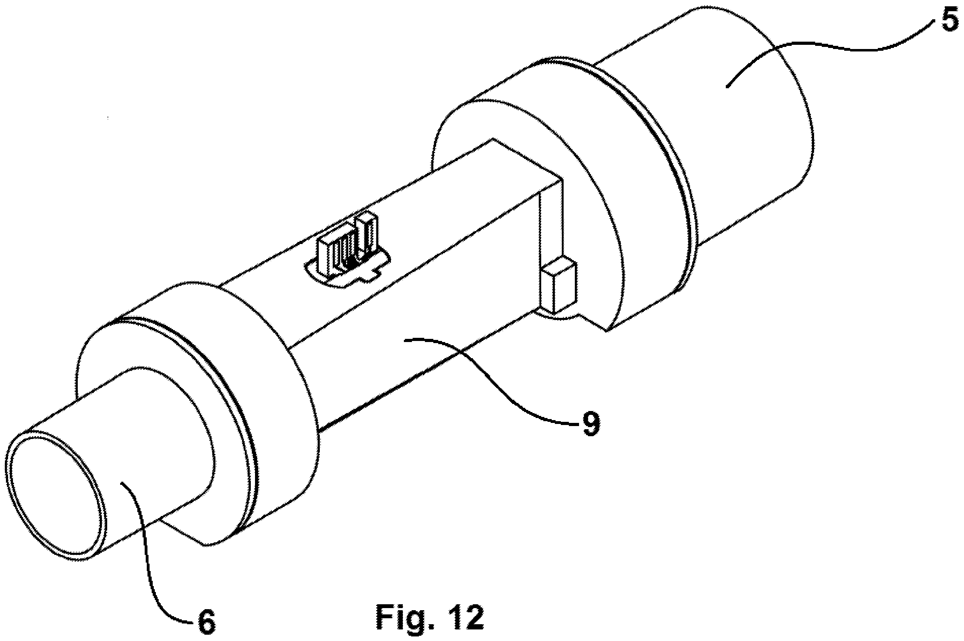

Figur 12 zeigt den Strömungstubus 2 mit den beiden Enden 5 und 6 und dem Sensorabschnitt 9. Im übrigen wird auf die Beschreibung des Strömungstubus 2 des ersten Ausführungsbeispiels verwiesen.

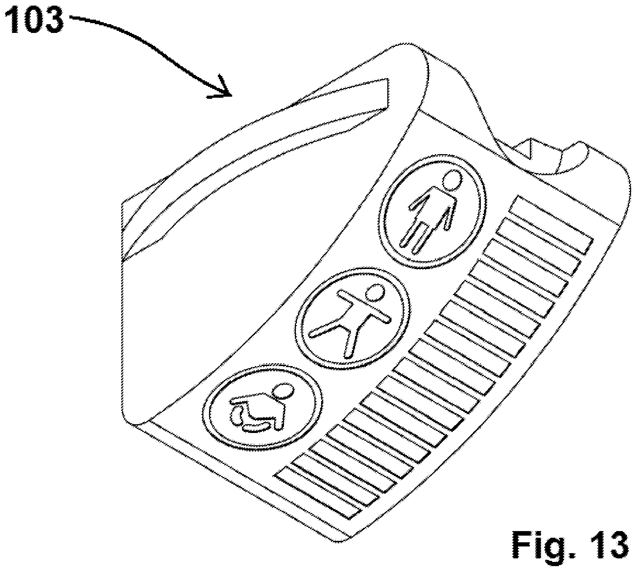

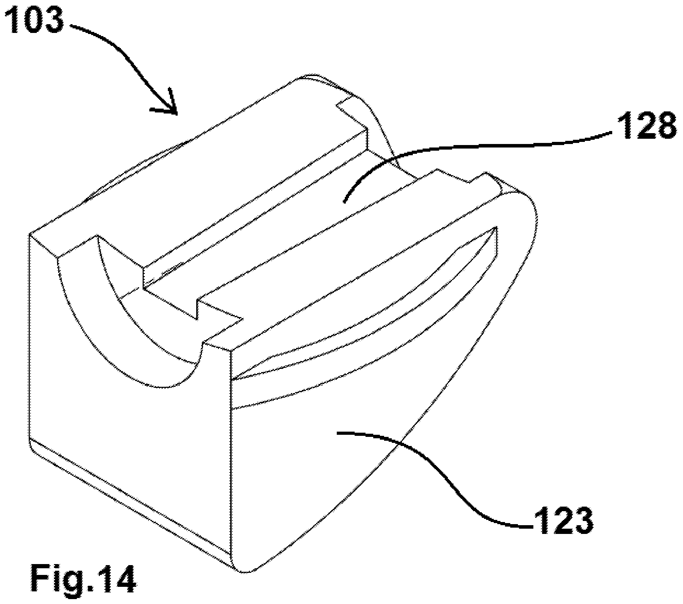

In den Figuren 13 und 14 ist das Aufsteckteil 103 dargestellt. In Figur 14 ist die Ausnehmung 128 in dem Gehäuse 123 erkennbar, in welche der Strömungstubus 2 eingesetzt wird.

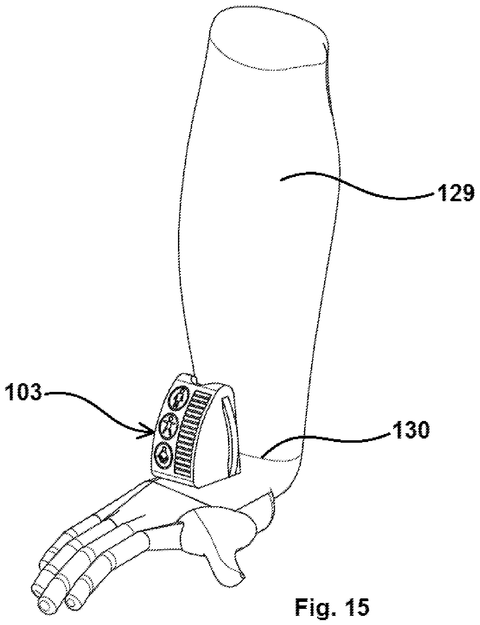

Figur 15 zeigt das Aufsteckteil 103, welches an einem Arm 129 eines Hilfeleistenden angeordnet ist. Hierzu wird das Aufsteckteil 103 mit einer Armbefestigungseinrichtung 130 in Form eines Bandes am Arm befestigt. In dem Aufsteckteil 103 ist ein in der Zeichnung nicht erkennbarer Beschleunigungssensor angeordnet.

Zum Messen des Masse- oder Volumenstroms einer Person wird die Vorrichtung 103 mit der Beatmungsmaske 40 auf Mund und Nase der Person aufgesetzt. Anhand der Eingabeeinrichtung 125 wird ausgewählt, zu welcher Gruppe von Personen die Person gehört. Der Strömungssensor in dem Strömungstubus erfasst den Masse- oder Volumenstrom der aus der Person ausströmenden Luft. Der erfasste Masse- oder Volumenstrom wird mit der Ausgabeeinrichtung 124 angezeigt. Ist der Masse- oder Volumenstrom zu klein, so führt der Hilfeleistende eine Herdruckmassage durch. Gegebenenfalls führt der Hilfeleistende über das Mundstück 41 Luft zu. Bei der Herzdruckmassage wird der Masse- oder Volumenstrom der aus der Person ausströmenden Luft erfasst. Wird kein Masse- oder Volumenstrom nachgewiesen, obwohl die Eindringtiefe erhöht wird, so ist dies ein Anzeichen für verlegte Atemwege. In diesem Fall kann der Hilfeleistende das Aufsteckteil 103 vom Strömungstubus 2 abnehmen und mit Hilfe der Armbefestigungseinrichtung 130 am Arm befestigen. Nun wird mithilfe des in das Gehäuse 123 integrierten Beschleunigungssensors die Eindringtiefe erfasst. Sind die Atemwege nicht verlegt, so kann kontinuierlich bei jeder Kompression des Brustkorbs der Person der Masse- oder Volumenstrom der aus der Person ausströmenden Luft erfasst und auf der Anzeigeeinrichtung angezeigt werden. Der Masse- oder Volumenstrom ist ein Maß für die Eindringtiefe bei der Herzdruckmassage. Über die Anzeigeeinrichtung erfährt der Hilfeleistende, ob die Eindringtiefe zu gering, ausreichend oder zu groß war. Er kann die Eindringtiefe bei der nächsten Kompression entsprechend anpassen.

Sämtliche Merkmale der Erfindung können sowohl einzeln als auch in beliebiger Kombination miteinander erfindungswesentlich sein.

### Bezugszahlen

- 1: Vorrichtung zur Atemspende in Notfällen
- 2: Strömungstubus
- 3: Aufsteckteil
- 4: Längsachse
- 5: Ende des Strömungstubus
- 6: Ende des Strömungstubus
- 7: Erste Stirnseite des Strömungstubus
- 8: Zweite Stirnseite des Strömungstubus
- 9: Sensorabschnitt
- 10: Sensor
- 10a: Sensorhalteeinrichtung
- 11: Sensorleiterplatte
- 12: Sensorhülse
- 13: Sensorhülse
- 14: Sensoröffnung
- 15: Seitenwand
- 16: Seitenwand
- 17: Seitenwand
- 18: Seitenwand
- 19: Erstes Filter
- 20: Zweites Filter
- 21: Muffe
- 22: Muffe
- 23: Gehäuse
- 24: Seitenwand
- 25: Seitenwand
- 26: Seitenwand
- 27: Leiterplatte
- 28: Leiterplatte
- 29: Steckerbuchse
- 30: Deckel
- 31: Äußeres Gehäuseteil
- 32: Äußeres Gehäuseteil
- 33: Griffelement
- 34: Griffelement
- 35: Griffmulde
- 36: Griffmulde
- 37:
- 38:
- 39:
- 40: Beatmungsmaske
- 41: Mundstück
- 42: Elastischer Rand
- 43: Band
- 44: Band
- 101: Vorrichtung zur Atemspende in Notfällen
- 103: Aufsteckteil
- 123: Gehäuse
- 124: Ausgabeeinrichtung
- 125: Eingabeeinrichtung
- 126: optisch aktive Oberfläche
- 127: Griffelement
- 128: Ausnehmung
- 129: Arm
- 130: Armbefestigungseinrichtung

## Patentansprüche

1. Vorrichtung zur Atemspende bei einer von einem medizinischen Notfall betroffenen Person
mit einer auf die Nasen- und Mundpartie der Person aufsetzbaren Beatmungsmaske (40),
mit einem Mundstück (41), durch welches Atemluft von einem Hilfeleistenden zuführbar ist,
mit einem zwischen der Beatmungsmaske (40) und dem Mundstück (41) angeordneten Strömungstubus (2), der einen durchgängigen Strömungskanal vom Mundstück (41) zur Beatmungsmaske (40) bildet,
mit mindestens einem in dem Strömungskanal des Strömungstubus (2) angeordneten Strömungssensor (10), mit dem ein Masse- oder Volumenstrom eines durch den Strömungskanal strömenden Gases erfassbar ist, mit einem Prozessor, der den mit dem Strömungssensor (10) erfassten Masse- oder Volumenstrom zu einem Ausgangssignal verarbeitet,
mit einer Ausgabeeinrichtung (124), welche das Ausgangssignal ausgibt, **dadurch gekennzeichnet, dass** der Prozessor als Eindringtiefe-Signal erzeugender Prozessor ausgebildet ist, der aus dem mit dem Strömungssensor (10) erfassten Masse- oder Volumenstrom bei einer Herzdruckmassage aufgrund einer Komprimierung des Brustkorbs bis zu einer Eindringtiefe aus Nase und/ oder Mund der Person ausströmenden und durch den Strömungskanal hindurchströmenden Luft ein für die Eindringtiefe charakteristisches Eindringtiefe-Signal erzeugt, und dass die Ausgabeeinrichtung (124) ausgebildet ist, das Eindringtiefe-Signal auszugeben.

2. Vorrichtung nach Anspruch 1, **dadurch gekennzeichnet, dass** sie zusätzlich zu dem Strömungssensor mit einem Vitalwert-Sensor ausgestattet ist, mittels dem ein Anteil an Sauerstoff und Kohlendioxid in der durch den Strömungskanal hindurchströmenden Luft erfassbar ist, und dass der Prozessor ausgebildet ist, um den erfassten Anteil an Sauerstoff und Kohlendioxid zu einem Ausgangssignal zu verarbeiten, welches mit der Ausgabeeinrichtung ausgegeben wird.

3. Vorrichtung nach Anspruch 1 oder 2, **dadurch gekennzeichnet, dass** die Ausgabeeinrichtung (124) eine optische Anzeigeeinrichtung aufweist.

4. Vorrichtung nach Anspruch 3 gekennzeichnet, dass die optische Anzeigeeinrichtung eine optisch aktive Oberfläche (126) aufweist, auf der das Ausgabesignal angezeigt wird, dass die optische aktive Oberfläche (126) eine nach außen gewölbte Oberfläche oder eine im wesentlichen ebene Oberfläche ist, und dass die ebene optisch aktive Oberfläche mit der Längsrichtung des Strömungstubus (2) einen Winkel von mehr als 0 ° und weniger 90 ° einschließt, wobei die Längsrichtung im wesentlichen der Strömungsrichtung der durch den Strömungstubus (2) strömenden Gase entspricht.

5. Vorrichtung nach Anspruch 3 oder 4, **dadurch gekennzeichnet, dass** die optische Anzeigeeinrichtung mehrere Licht emittierende Dioden LEDs aufweist.

6. Vorrichtung nach einem der vorhergehenden Ansprüche, **dadurch gekennzeichnet, dass** die Ausgabeeinrichtung eine akustische Ausgabeeinrichtung aufweist.

7. Vorrichtung nach einem der vorhergehenden Ansprüche, **dadurch gekennzeichnet, dass** sie mit einer Schnittstelle ausgestattet ist, über die Daten betreffend einen durch den Strömungssensor (10) erfassten Masse- oder Volumenstrom oder daraus abgeleitete Daten an ein externes Gerät zur Notfallversorgung, an ein medizinisches Gerät, an einen Computer und/ oder an eine mobiles Telekommunikationsgerät ausgebbar sind.

8. Vorrichtung nach einem der vorhergehenden Ansprüche, **dadurch gekennzeichnet, dass** zwischen dem Mundstück (41) und dem Strömungssensor (10) in dem Strömungskanal ein Filter (19) und/ oder zwischen der Beatmungsmaske (40) und dem Strömungssensor (10) in dem Strömungskanal ein Filter (20) angeordnet ist.

9. Vorrichtung nach Anspruch 1, **dadurch gekennzeichnet, dass** die Ausgabeeinrichtung (124) ausgebildet ist, anzuzeigen, ob der erfasste Masse- oder Volumenstrom oder das daraus abgeleitete Eindringtiefe-Signal oder eine sonstige aus dem Masse- oder Volumenstrom abgeleitete Größe innerhalb eines für eine Reanimation vorgegebenen Bereichs liegt.

10. Vorrichtung nach einem der vorhergehenden Ansprüche, **dadurch gekennzeichnet, dass** sie mit einem Aufsteckteil (3, 103) ausgestattet ist, welches lösbar mit dem Strömungstubus (2) verbindbar ist, und dass in dem Aufsteckteil (3, 103) der Prozessor und die Ausgabeeinrichtung (124) angeordnet sind.

11. Vorrichtung nach Anspruch 10, **dadurch gekennzeichnet, dass** das Aufsteckteil (103) eine Armbefestigungseinrichtung (130) aufweist, mit der es an einem Arm eines Hilfeleistenden befestigbar ist, dass das Aufsteckteil (103) einen Beschleunigungssensor aufweist, welcher eine Beschleunigung des Arms bei einer Herzdruckmassage erfasst.

12. Vorrichtung nach Anspruch 11, **dadurch gekennzeichnet, dass** der Prozessor ausgebildet ist, aus der mit dem Beschleunigungssenor erfassten Beschleunigung ein Ausgangssignal zu erzeugen, welches mit der Ausgabeeinrichtung (124) ausgebbar ist.

13. Vorrichtung nach einem der vorhergehenden Ansprüche, **dadurch gekennzeichnet, dass** sie einen zusätzlichen zweiten Vitalwert-Sensor aufweist, der als Vitalwert der Person die Druckdifferenz in dem Strömungskanal erfasst.

## Claims

1. Device for artificial respiration of a person in a medical emergency situation
with a respiratory mask (40) which can be placed on the nose and mouth section of the person,
with a mouthpiece (41), through which respiratory air can be supplied by an aider,
with a flow tube (2) disposed between the respiratory mask (40) and the mouthpiece (41), which flow tube (2) forms a continuous flow channel from the mouthpiece (41) to the respiratory mask (40),
at least one flow sensor (10) disposed in the flow channel of the flow tube (2), which flow sensor (10) can determine a mass or volumetric flow of a gas flowing through the flow channel,
with a processor that processes the mass or volumetric flow registered by the flow sensor (10) into an output signal,
with an output device (124), which emits the output signal, **characterized in that**,
the processor is designed as a processor generating a penetration depth signal, which processor generates a characteristic penetration depth signal for the penetration depth from the mass or volumetric flow registered by the flow sensor (10) during a cardiac massage as a result of a compression of the thorax down to a penetration depth, for air flowing out of the nose and/or mouth of the person and through the flow channel, and wherein the output device (124) is designed to emit the penetration depth signal.

2. Device according to claim 1, wherein in addition to the flow sensor it is equipped with a vital signs sensor, by means of which a proportion of oxygen and carbon dioxide in the air flowing through the flow channel can be registered, and wherein the processor is designed to process the registered proportion of oxygen and carbon dioxide into an output signal, which is emitted by the output device.

3. Device according to claim 1, wherein the output device (124) exhibits an optical display device.

4. Device according to claim 3, wherein the optical display device exhibits an optically active surface (126) on which the output signal is displayed, wherein the optically active surface (126) is a convex surface or essentially a flat surface, and wherein the flat optically active surface includes an angle of more than 0° and less than 90° with the longitudinal direction of the flow tube (2), where the longitudinal direction essentially corresponds to the direction of flow of the gases flowing through the flow tube (2).

5. Device according to claim 3 or 4, wherein the optical display device exhibits several light-emitting diodes (LEDs).

6. Device according to one of the previous claims, wherein the output device has an acoustic output device.

7. Device according to one of the previous claims, wherein it is equipped with an interface over which data concerning a mass or volumetric flow registered by the flow sensor (10) or data derived therefrom can be emitted to an external device for emergency care, to a Medical device, to a computer and/or to a mobile telecommunications device.

8. Device according to one of the previous claims, wherein a filter (19) is arranged in the flow channel between the mouthpiece (41) and the flow sensor (10) and/or a filter (20) in the flow channel between the respiratory mask (40) and the flow sensor (10).

9. Device according to claim 1, wherein the output device (124) is designed to indicate whether the mass or volumetric flow registered or the penetration depth signal derived from it or another parameter derived from the mass or volumetric flow lies within a specified range for resuscitation.

10. Device according to one of the previous claims, wherein it is equipped with a push-on part (3, 103) that can be connected detachably to the flow tube (2), and wherein the processor and the output device (124) are arranged in the push-on part (3, 103).

11. Device according to claim 10, wherein the push-on part (103) exhibits an arm fastening device (130) with which it can be fastened to an arm of an aider, and wherein the push-on part (103) exhibits an acceleration sensor, which registers an acceleration of the arm during a cardiac massage.

12. Device according to claim 11, wherein the processor is designed to generate an output signal from the acceleration registered by the acceleration signal, which output signal can be emitted by the output device (124).

13. Device according to one of the previous claims, wherein it exhibits an additional second vital signs sensor that registers the pressure difference in the flow channel as a vital sign of the person.

## Revendications

1. Dispositif d'assistance respiratoire pour une personne se trouvant dans une situation d'urgence médicale
comportant un masque de réanimation (40), qui peut être appliqué sur le nez et la bouche de la personne,
une partie buccale (41), permettant de recevoir l'air insufflé par un secouriste, un tube d'écoulement (2), situé entre le masque de réanimation (40) et la partie buccale (41), qui constitue un canal d'écoulement continu entre la pièce buccale (41) et le masque (40),
au moins un capteur de flux (10), situé dans le canal d'écoulement du tube d'écoulement (2), permettant de mesurer le débit massique ou volumétrique d'un gaz traversant le canal d'écoulement,
un processeur qui transforme le débit massique ou volumétrique relevé par le capteur de flux (10) en signal de sortie,
un dispositif de sortie (124) qui sort le signal
**caractérisé par le fait**
**que** le processeur est conçu comme générateur de signal de profondeur de pénétration et produit en tant que tel un signal caractéristique de la profondeur de pénétration à partir du débit massique ou volumétrique relevé avec le capteur de flux (10) lors d'un massage cardiaque externe, comme résultat de la compression de la cage thoracique jusqu'à une certaine profondeur de pénétration, sur la base de l'air sortant du nez ou de la bouche de la personne et traversant le canal d'écoulement, et que le dispositif de sortie (124) est conçu pour la sortie du signal de profondeur de pénétration.

2. Dispositif selon la revendication 1, **caractérisé par le fait qu'**il possède en plus du capteur de flux un capteur de valeur vitale, avec lequel une part d'oxygène et de dioxyde de carbone de l'air qui traverse le canal d'écoulement est mesurable, et **caractérisé par le fait que** le processeur est conçu de manière à traiter la part mesurée d'oxygène et de dioxyde de carbone en un signal sorti par le dispositif de sortie.

3. Dispositif selon la revendication 1 ou 2, **caractérisé par le fait que** le dispositif de sortie (124) possède un dispositif d'affichage optique.

4. Dispositif selon la revendication 3, **caractérisé par le fait que** le dispositif d'affichage optique présente une surface optiquement active (126), sur laquelle le signal de sortie est affiché, **par le fait que** la surface optiquement active (126) est une surface courbée vers l'extérieur ou une surface essentiellement plane, et que cette surface plane forme avec l'axe longitudinal du tube d'écoulement (2) un angle supérieur à 0° et inférieur à 90°, l'axe longitudinal correspondant pour l'essentiel à la direction du flux des gaz traversant le tube d'écoulement (2).

5. Dispositif selon la revendication 3 ou 4, **caractérisé par le fait que** le dispositif d'affichage optique comporte plusieurs diodes électroluminescentes (LED).

6. Dispositif selon l'une des revendications précédentes, **caractérisé par le fait que** le dispositif de sortie intègre un dispositif d'émission acoustique.

7. Dispositif selon l'une des revendications précédentes, **caractérisé par le fait qu'**il est pourvu d'une interface à travers laquelle le débit massique ou volumétrique relevé par le capteur de flux (10) ou les données qui en sont dérivées peuvent être sortis sur un appareil externe utilisé pour les soins d'urgence, sur un appareil médical, sur un ordinateur ou sur un appareil de télécommunication mobile.

8. Dispositif selon l'une des revendications précédentes, **caractérisé par le fait qu'**un filtre (19) se trouve entre la pièce buccale (41) et le capteur de flux (10) dans le canal d'écoulement et/ou un filtre (20) entre le masque de réanimation (40) et le capteur de flux (10).

9. Dispositif selon la revendication 1, **caractérisé par le fait que** le dispositif de sortie (124) est conçu pour indiquer si le débit massique ou volumétrique relevé ou le signal de profondeur de pénétration qui en est dérivé ou une autre grandeur déterminée à partir du débit massique ou volumétrique, se situe dans une plage définie pour la réanimation.

10. Dispositif selon l'une des revendications précédentes, **caractérisé par le fait qu'**il est muni d'une unité de base (3, 103), qui se connecte de façon détachable au tube d'écoulement (2), et **par le fait que** le processeur et le dispositif de sortie (124) sont intégrés dans cette unité de base (3, 103).

11. Dispositif selon la revendication 10, **caractérisé par le fait que** l'unité de base (103) est pourvu d'un dispositif de fixation au bras (130), avec lequel elle peut être fixée au bras d'un secouriste, que l'unité de base (103) est pourvue d'un capteur d'accélération qui mesure l'accélération du bras lors d'un massage cardiaque externe.

12. Dispositif selon la revendication 11, **caractérisé par le fait que** le processeur est conçu pour générer à partir de l'accélération saisie avec le capteur d'accélération un signal pouvant être sorti par le dispositif de sortie (124).

13. Dispositif selon l'une des revendications précédentes, **caractérisé par le fait qu'**il présente un deuxième capteur de valeur vitale, qui relève la différence de pression dans le canal d'écoulement comme valeur vitale de la personne.
